# EUROPÄISCHE PATENTSCHRIFT

(11) **EP 0 754 094 B1**
(45) Veröffentlichungstag und Bekanntmachung des Hinweises auf die Patenterteilung: **13.10.1999**
(21) Anmeldenummer: 95909691.8
(22) Anmeldetag: 09.02.1995
(51) Int. Cl.: B05B 11/00, B05B 11/02

(54) **AUSTRAGVORRICHTUNG FÜR FLIESSFÄHIGE MEDIEN, INSBESONDERE FÜR DEN AUSTRAG IN NUR EINEM HUB**
DISCHARGE DEVICE FOR FREE-FLOWING LIQUIDS, IN PARTICULAR FOR DISCHARGE IN ONLY ONE STROKE
DISPOSITIF VERSEUR POUR SUBSTANCES COULANTES, NOTAMMENT POUR VERSEMENT PAR COURSE UNIQUE DU CORPS DE POMPE

(30) Priorität: 08.04.1994 DE 4412041
(43) Veröffentlichungstag der Anmeldung: 22.01.1997
(73) Patentinhaber: Ing. Erich Pfeiffer GmbH, 78315 Radolfzell (DE)
(72) Erfinder: FUCHS, Karl-Heinz, D-78315 Radolfzell (DE)
(74) Vertreter: Ruff, Michael
(86) Internationale Anmeldenummer: EP9500457
(87) Internationale Veröffentlichungsnummer: WO9527568

(56) Entgegenhaltungen:
- EP-A- 0 546 607
- FR-A- 1 535 293

## Beschreibung

### Anwendungsgebiet und Stand der Technik

Die Erfindung betrifft eine Austragvorrichtung nach dem Oberbegriff des Anspruchs 1.

In der EP-PS 0 311 863 A der Anmelderin und der EP 0546 607 A (vgl. Oberbegriff des Anspruchs 1) ist beschrieben, daß der Pumpenzylinder und ein nach Art einer Schnappverriegelung ausgebildeter federnder Anschlag so zusammenwirken, daß vor dem Austrag eines Hubes bzw. eines Teilhubes ein bestimmter Betätigungsdruck von dem Bedienenden aufgebracht werden muß, so daß nach Überwindung dieses Druckpunktes der Austrag der Flüssigkeit mit einer bestimmten Mindestkraft und -geschwindigkeit erfolgt.

Diese Ausbildung stellt sicher, daß z.B. bei Zerstäubung des Mediums der Druck vom ersten Moment an zur Zerstäubung ausreicht und daß die Pumpe bis zu ihrem Ende betätigt wird, also den vollen Hub ausführt und den gesamten Inhalt ihres Medienspeichers, der gleichzeitig den Pumpenzylinder bildet, in einem oder zwei Hüben ausgibt. Solche Einmal- oder Zweimal-Dosierer sind bedeutsam für die Ausgabe von Medikamenten, die bzgl. der Dosierung, Kontaminierung, Konservierung oder weiterer Kriterien besonders kritisch sind.

Aus der FR-A-1 535 293 ist eine Zerstäuberpumpe bekanntgeworden, die eine übliche, zur Durchführung zahlreicher Hübe ausgebildete Schubkolbenpumpe aufweist, deren Pumpenzylinder durch einen Grundkörper auf dem Behälterhals festgehalten ist, der nach Art einer Überwurfmutter aufgeschraubt ist. Auf die Kolbenstange der Schubkolbenpumpe ist ein Sprühkopf mit einem Kugelventil aufgesteckt, der über Sollbruchstellen mit einem Außenring verbunden ist, der auf den Aufschraubkörper aufsetzbar ist. Bei der ersten Betätigung des Sprühkopfes werden die Sollbruchstellen durchbrochen, und die Pumpe kann dann in zahlreichen Hüben betätigt werden. Dabei können allerdings Verhakungen zwischen den beiden durch die Sollbruchstellen miteinander verbundenen Teile auftreten.

Ferner ist es in der WO 92/00812 der Anmelderin offenbart, Medienspeicher für nur einen Austraghub, die mit einem gleichzeitig als Kolben dienenden Stopfen verschlossen sind, zu benutzen, wobei der Stopfen zur Betätigung von einer Nadel durchstoßen wird. Der Medienspeicher ist dabei in einer Hülse aufgenommen, die äußere Vorsprünge hat, die mit entsprechenden Vorsprüngen am Inneren eines Gehäuse-Grundkörpers nach Art einer Schnappbefestigung zusammenwirken. Beim Betätigen muß zuerst die Ruhereibung überwunden werden, bevor diese Wülste auf den Flächen, an denen sie anliegen, zum Gleiten kommen, so daß dabei ein Druckpunkt zu überwinden ist.

### Aufgabe und Lösung

Aufgabe der Erfindung ist es, eine derartige Austragvorrichtung weiterzuentwickeln, insbesondere, um eine versehentliche Doppelbenutzung oder unvollständige Betätigung sowie sich daraus ergebende Kontaminations- oder Dosierungsprobleme zu vermeiden.

Diese Aufgabe wird gemäß der Erfindung durch die Merkmale des Anspruchs 1 gelöst.

Die Materialbrücke kann bei einer Ausführungsform der Erfindung zwischen wenigstens einem mit dem Pumpenzylinder bewegbaren Pumpenabschnitt und einem mit dem Grundkörper verbundenen Gehäuseabschnitt gebildet sein. Dabei kann die Materialbrücke vorteilhaft durch einstückige Ausbildung von Gehäuse- und Pumpenabschnitt gebildet sein.

Es wird also eine Sollbruchstelle zwischen zwei gegeneinander bewegbaren Teilen der Austragvorrichtung geschaffen, deren Unverletztheit ein sicheres Zeichen dafür ist, daß die Charge im Medienspeicher ungeöffnet und unverbraucht ist. Der Benutzer kann dies beispielsweise optisch oder auch durch leichtes Drehen an dem Betätigungsteil der Pumpe feststellen. Zur Erleichterung der Überprüfung könnten Fensterausschnitte vorgesehen oder Teile der Pumpe aus transparentem Material hergestellt sein. Es ist auch möglich, einen Kunststoff zu verwenden, der sich bei der Sollbruchverformung verfärbt. Auch Markierungen an dem bewegbaren Abschnitt und dem Gehäuse wären möglich. Die Materialbrücke ist vorzugsweise zwischen einer den als Medienspeicher dienenden Pumpenzylinder haltenden und ggf. teilweise aufnehmenden Betätigungshülse und einem Innenabschnitt eines mit Betätigungsschultern versehenen Basisgehäuseteils ausgebildet. Die Betätigungsschultern dienen dabei als Stütze für zwei Finger, während mit dem Daumen auf diese Betätigungshülse gedrückt wird. Dabei kann genügend Kraft aufgebracht werden, um den Materialabschnitt abzuscheren oder abzureißen. Durch die dann aufgebrachte Kraft wird auch gleichzeitig eine ausreichende Betätigungskraft für eine sichere Betätigung der Pumpe erzeugt.

Die Materialbrücken können z.B. am Außenumfang der Betätigungshülse vorgesehen und zwischen dieser und einem die Betätigungshülse umgebenden Ring verlaufen, der seinerseits an dem gegenüberliegenden Teil, also am Gehäuse, durch Einschnappen festgelegt ist. Dies ermöglicht es, die Betätigungshülse getrennt vom Gehäuse herzustellen. Die Materialbrücken könnten aber auch am Gehäuse angeformt sein, und das ringförmige Befestigungselement könnte an einem demgegenüber beweglichen Teil, dem Pumpenzylinder oder der Betätigungshülse, durch Einrasten oder auf andere Weise befestigt sein.

Um den Pumpenzylinder, der das Medium enthält und der meist aus Gründen der Diffusionssicherheit und Verträglichkeit aus Glas besteht, in die Austragvorrichtung einsetzen zu können, ist vorzugsweise das Basisgehäuseteil mit einem die Pumpe zumindest teilweise aufnehmenden und die Austragöffnung aufweisenden Stutzenabschnitt, beispielsweise durch verrastenden Eingriff verbunden.

Vorteilhaft sind mehrere Materialbrücken gleichmäßig über den Umfang der miteinander verbundenen Teile verteilt, um eine Verkantung bei der Betätigung zu vermeiden. Diese Materialbrücken können vorteilhaft in einem Bereich angeordnet sein, in dem eines der Teile, beispielsweise die Betätigungshülse, ihre Endkante hat und das andere Betätigungsteil oberhalb, aber etwas außerhalb davon beginnt. Es bildet sich dort praktisch eine Trennebene zwischen miteinander zusammenwirkenden Werkzeugteilen im Kunststoffspritzguß, zwischen denen die Materialbrücken nach Art eines Spritzgrates, jedoch mit kontrollierter Sollbruchkraft, stehen bleiben.

Ferner könnte, um ein sicheres Durchtrennen der Materialbrücken mit einer vorgegebenen Kraft zu ermöglichen, an einem bei der Betätigung der Pumpe zusammenwirkenden Teile, vorzugsweise am Grundkörper, eine Schneidkante zum Durchtrennen der Materialbrücke vorgesehen sein. Dies kann beispielsweise eine Innenkante des mit dem Basis-Gehäuseteils verbundenen Stutzenabschnittes sein. Während es ohne eine solche Schneidkante vorteilhaft ist, diese Sollbruchstelle vorwiegend auf Scherung zu beanspruchen, könnte in diesem Falle, der auch bei der Verwendung von Materialien mit hoher Bruchdehnung vorteilhaft ist, auch eine stegartige Ausformung der Materialbrücken zweckmäßig sein.

Gemäß einer anderen Ausführungsform kann die Originalitätssicherung im Bereich eines den Pumpenkolben bildenden Kolbenstopfen durchstoßenden Stößels durch eine einen erhöhten Widerstand vor dem Durchstoßen erzeugende Stößelgestaltung gebildet sein. Hierbei wird also bewußt dem Stößel ein kontrollierter Widerstand entgegengesetzt, bevor er mit dem Durchstoßen den Beginn des Pumphubes einleitet. Im Gegensatz zu einer Nadelausbildung, die hauptsächlich dazu ausgelegt ist, den Kolbenstopfen mit so geringer Kraft wie möglich zu durchstoßen, wird hier also ganz bewußt ein Widerstand in Kauf genommen. Dabei sollte allerdings darauf geachtet werden, daß das Durchstoßen ohne Ablösung von Teilen des Kolbenstopfens geschieht, damit diese nicht in das Medium geraten und die Austrittsdüse verstopfen können.

Der Stößel kann beispielsweise aus Kunststoff geformt sein, und zwar mit einem sonstigen aktiven Pumpenteil zusammen, was z.B. den Mehraufwand erübrigt, den das Einsetzen einer Stahlnadel machen würde. Obwohl bei den relativ großen Abmessungen, die der Stößel aufgrund seiner Funktion haben kann, die Ausbildung eines Innenkanals in ihm nicht so problematisch wäre, ist es jedoch herstellungsmäßig viel einfacher, wenn der Stößel seitliche Leitkanäle für das Medium aufweist, in dem er beispielsweise einen kreuzartigen Querschnitt hat. Die Kanäle, die das Medium dann durch den Kolbenstopfen leiten, werden dabei von außen von ihm selbst abgeschlossen. Dabei ist es vorteilhaft, wenn der Boden des Kolbenstopfens, der durchstoßen wird, am Ende einer Vertiefung liegt, die den Stößel umschließt und somit die Kanäle abgrenzt. Eine obere Fläche des Kolbenstopfens kann mit einem entsprechenden Teil an der Pumpe zusammenwirken, um auch dort eine Abdichtung nach außen zu schaffen.

Bei dem Eindringen des Stößels in diese Vertiefung kann der notwendige Gegendruck erzeugt werden, der für die Originalitätssicherung notwendig ist und über die vorher beschriebenen Markierungen oder andere Maßnahmen kann abgelesen werden, ob die Pumpe bereits betätigt war oder nicht.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird.

### Figurenkurzbeschreibung

Ausführungsbeispiele der Erfindung sind in den Zeichnungen dargestellt und werden im folgenden näher erläutert. In den Zeichnungen zeigen:
- Fig. 1: einen Längsschnitt durch eine Austragvorrichtung mit Originalitätssicherung,
- Fig. 2: eine Ansicht in Richtung des Pfeiles II in Fig. 1 gesehen,
- Fig. 3: ein Detail aus Fig. 2, jedoch im Schnitt dargestellt,
- Fig. 4: einen Schnitt nach der Linie IV in Fig. 2,
- Fig. 5: eine abgewandelte Ausführungsform der Austragvorrichtung nach Fig. 1 im Längsschnitt,
- Fig. 6: ein Detail aus Fig. 5,
- Fig. 7: eine Ansicht in Richtung des Pfeils VII in Fig. 5,
- Fig. 8: einen Teil-Längsschnitt durch eine Ausführungsform mit angeformtem Originalitätssicherungsring.

### Beschreibung der Ausführungsbeispiele

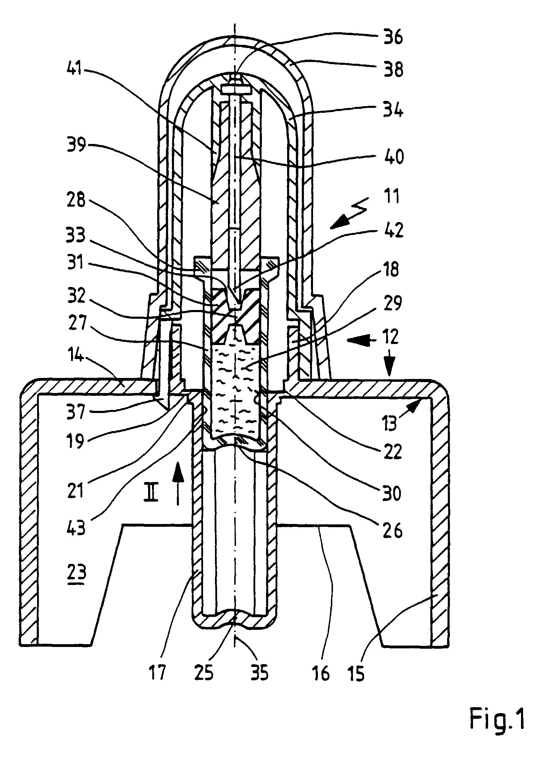

Die in Fig. 1 dargestellte Austragvorrichtung 11 weist einen Grundkörper 12 auf, der zweiteilig ausgebildet ist. Er enthält ein Basisgehäuseteil 13, das eine epaulettenartige Form mit zwei Betätigungsschultern 14 und einem daran anschließenden Mantel 15 hat. Das Basisgehäuseteil ist flach oder flachoval und hat seine größte Erstreckung in der Zeichenebene, während es quer dazu geringere Abmessungen hat. Der Mantel 15 ist an den in Fig. 1 rechten und linken Seiten länger und hat an seinen beiden etwa parallel zur Zeichenebene verlaufenen Seiten einen Ausschnitt 16. Es ist nach unten offen.

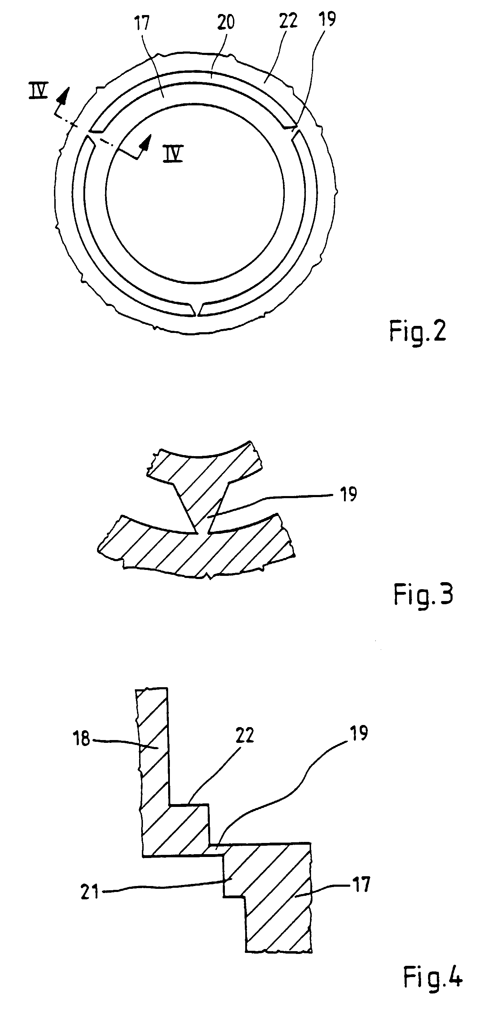

An das Basisgehäuseteil 13 ist einstückig eine Betätigungshülse 17 angespritzt, und zwar an der unteren inneren Seite eines vom Basisgehäuseteil 13 nach oben ragenden Stutzens 18. Die einstückige Verbindung zwischen Basisgehäuseteil 13 und Betätigungshülse 17 erfolgt über Materialbrücken 19, wie sie in den Fig. 2 bis 4 dargestellt sind. Der an sich vorgesehene Spalt 20 zwischen der Außenkante eines oberen Flansches 21 der Betätigungshülse und einem inneren Flanschvorsprung 22 im Inneren des Stutzens 18 wird im dargestellten Beispiel durch drei dünne Verbindungsstege überbrückt, die eine Dreiecksform haben und mit ihrer Spitze am Steg 22 angeformt sind. Sie bilden Sollbruchstellen. Herstellungsmäßig können sie durch entsprechende geringfügige Ausnehmungen, die kaum die Dimensionen eines normalen Spritzgrates übersteigen, an der Trennfläche zwischen zwei Kunststoffspritzgußformteilen entstehen, die einerseits den Raum 23 im Inneren des Mantels 15 und andererseits den Raum im Stutzen 18 formen. Dementsprechend liegen die drei gleichmäßig über den Umfang verteilten Materialbrücken 19 in einer durch die Innen- oder Unterseite der Betätigungsschultern 14 einerseits und durch die Oberkante des Flansches 21 andererseits gebildeten Ebene.

Die Betätigungshülse 17 hat innere Rippen 24, die etwa 3/4 der Länge der Hülse, von ihrem Boden 25 aus gesehen, einnehmen. Auf ihnen stützt sich der Boden 26 eines Pumpenzylinders 27 ab, der die Form einer etwa zylindrischen, unten geschlossen Hülse aus Glas mit einem oberen Seitenflansch 28 hat. Er bildet gleichzeitig den Speicher für das auszutragende Medium 29 und den Pumpenzylinder. Seine innere Mantelfläche bildet demnach die Kolbenlaufbahn 30 für einen Kolben 31, der in Form eines Kolbenstopfens aus Gummi oder einem ähnlichen elastischen Material ausgebildet ist. Er hat im Längsschnitt eine H-Form und ist ein dickwandiges Rohr, dessen Außenmantel die Kolbenlaufflächen bildet, mit einem mittleren, das Rohrinnere quer verschließenden Verschlußsteg 32, so daß sich oben und unten, anschließend an den Verschlußsteg 32 Vertiefungen 33 bilden, die etwas konisch ausgebildet sein können.

Der Kolbenstopfen 31 sitzt auf dem hermetisch abgeschlossenen Medium 29. Die Abfüllung erfolgt mit oder ohne Lufteinschlüsse. In den oberen Teil der Betätigungshülse ist der Pumpenzylinder mit leichter Pressung eingesetzt. Er erstreckt sich mit seinem größten Teil über die Betätigungsschultern 14 hinweg durch den Stutzen 18 hindurch in einen Stutzenabschnitt 34 hinein, der auch als Adapter bezeichnet und ausgebildet werden kann. Es handelt sich dabei um einen fingerartigen Abschnitt, der einen in Richtung der Pumpenachse 35 verlaufenden, im wesentlichen zylindrischen oder leicht konischen Schaft mit einer oberen kugligen Rundung hat. In der Mitte dieser Rundung ist die Austragöffnung 36 in Form einer üblichen Sprühdüse vorgesehen. Die Austragöffnung könnte auch für eine andere Ausbringungsform, beispielsweise für die bloße dosierte Ausgabe eines flüssigen oder pastösen Mediums, vorgesehen sein oder auch andere Formen haben, um beispielsweise die Ausgabe der Form irgendeiner Körperöffnung besser anzupassen. Aus diesem Grunde ist es auch vorteilhaft, daß der Stutzenabschnitt 34 gesondert von dem Basisgehäuseteil hergestellt und auf diesem durch eine Schnappbefestigung 37 mit z.B. drei in Öffnungen eingreifenden, mit einem widerhakenartigen Kopf versehenen Laschen festzulegen ist, wobei sich der Stutzenabschnitt auf dem Stutzen 18 zentriert. Um ein Abnehmen des Stutzenabschnittes 34 zu verhindern, kann die Schnappbefestigung selbstsperrend ausgebildet sein. Eine Schutzhülse 38 schützt den Stutzenabschnitt vor Verschmutzungen.

Im Inneren des Stutzenabschnittes verläuft ein Kolbenstangenabschnitt 39, der einen inneren Austragkanal 40 aufweist und in eine an das obere Ende des Stutzenabschnitts 34 nach innen angeformte Hülse 41 eingesteckt ist. In diesem Kolbenstangenabschnitt 39 ist ein Stößel 42 in Form einer unten schräg abgeschnittenen hohlen Stahlnadel eingesetzt, beispielsweise durch Einspritzen oder Einpressen in eine mit entsprechenden Halterrippen versehene Öffnung.

Bis auf die Stahlnadel 42, den aus einem gummiartigen Material bestehenden Kolbenstopfen 31 und den aus Glas bestehenden Pumpenzylinder/Medienspeicher bestehen alle Teile der Pumpe aus Kunststoffspritzguß.

Bei der Herstellung wird in den das Basisgehäuseteil 13 und die Betätigungshülse 17 umfassenden Bauteil von oben her in die in der Betätigungshülse 17 gebildete Aufnahme 43 der Pumpenzylinder 27 eingesetzt. In diesem ist das Medium 29, durch den Kolbenstopfen 31 dicht abgeschlossen, enthalten.

Danach wird der Stutzenabschnitt, an dem der Kolbenstangenabschnitt und der Stößel 42 vormontiert sind, aufgesetzt, wobei er in das Basisgehäuseteil eingreift und sich über die Schnappbefestigung 37 festlegt. Nach Aufsetzen der Schutzhülse 38 ist damit die Austragvorrichtung montiert.

Zur Benutzung wird die Austragvorrichtung nach Abnehmen der Schutzhülse 38 vom Benutzer zwischen drei Finger genommen, wobei zwei Finger auf den Schultern 14 liegen und der Daumen auf dem Boden 25 der Betätigungshülse 17. Der Daumen greift dabei in den fensterartigen Ausschnitt 16 ein und hat so ausreichenden Betätigungsspielraum. Die Austragöffnung 36 wird auf die entsprechende Stelle gerichtet und durch einen kräftigen Druck auf die Betätigungshülse der Betätigungsdruck so weit aufgebaut, bis die durch die Materialbrücken 19 gebildeten Sollbruchstellen reißen bzw. abscheren und die Betätigungshülse zusammen mit dem Pumpenzylinder aufwärts bewegt werden kann. Durch den vorherigen starken Druckaufbau geschieht das nun mit großer Geschwindigkeit, die für eine zügige Durchführung des nun folgenden Ausgabehubes sorgt. Dabei wird der Pumpenzylinder nach oben gegen den Stößel 42 bewegt, so daß dieser den Mittelsteg 32 des Kolbenstopfens durchstößt, infolge der Elastizität des Kolbenstopfenmaterials jedoch sofort an der Außenfläche wieder abgedichtet ist. Nur durch den inneren Kanal des als Hohlnadel ausgebildeten Stößels kann das Medium um nach oben durch den Austragkanal 40 und die Austragöffnung 36 hinaus entweichen und wird dort versprüht oder entsprechend dosiert ausgegeben. Die Unterkante des Kolbenstangenabschnittes 39 kann sich dabei auf die obere Fläche des Kolbenstopfens 31 legen und dadurch auch eine direkte Druckverbindung zu dem Kolben herstellen, der nun längs der Kolbenlaufbahn 30 nach unten läuft und das Medium zur Austragöffnung hinausfördert. Die Nadel sollte in ihrer Länge so bemessen sein, daß sie nicht über die untere Begrenzung des Kolbenstopfens 31 hinausragt, so daß eine nahezu vollständige Ausgabe des unter Umständen sehr teueren Mediums gewährleistet ist.

Es ist also zu erkennen, daß mit dieser Austragvorrichtung eine Möglichkeit geschaffen ist, empfindliche und teuere Materialien gezielt und genau dosiert auszugeben. Durch die mit Materialzerstörung arbeitende Originalitätssicherung ist jederzeit die Unversehrtheit zu überprüfen und es ist sichergestellt, daß das Medium gänzlich und mit einem ausreichenden Betätigungsdruck ausgegeben wird. Die Vorrichtung ist einfach herzustellen und zu montieren sowie leicht unterschiedlichen Gegebenheiten, beispielsweise durch verschiedene Adapterformen, anzupassen. Nach der Betätigung ist die aus Pumpenzylinder 27 und Betätigungshülse bestehende Einheit lose und kann ggf. auch nach unten herausgezogen werden. Der Pumpenzylinder 27 mit dem Kolbenstopfen 31 kann entfernt werden, so daß der übrige Teil, bis auf die winzige Stahlnadel, artenrein aus ggf. einem Kunststofftyp besteht und entsprechend entsorgt werden kann.

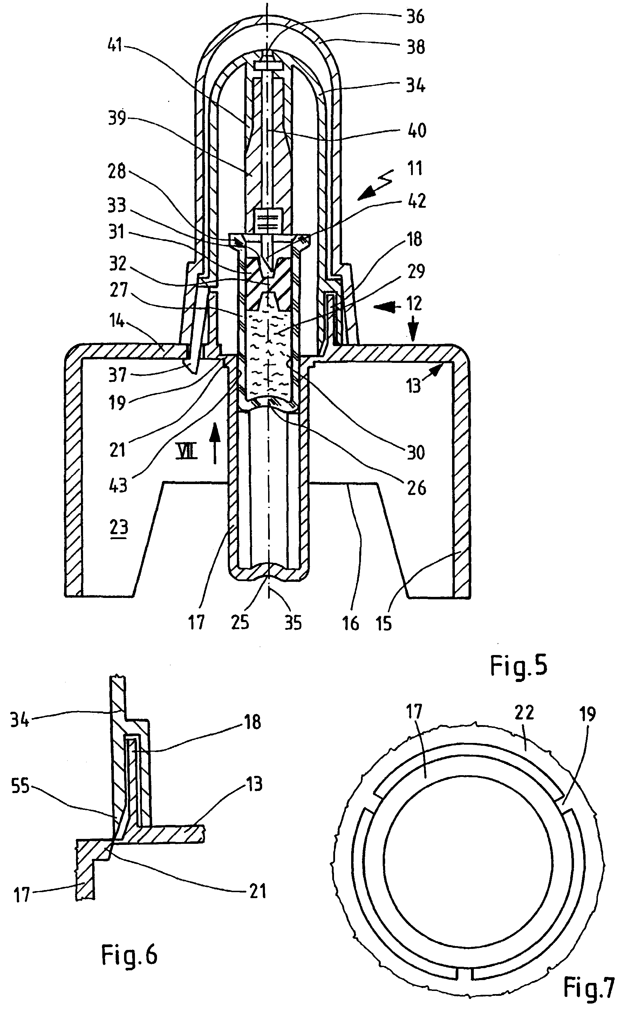

Bei der Ausführungsform nach Fig. 5 sind alle Teile und Funktionen identisch mit denen nach Fig. 1 bis 4 mit folgenden Ausnahmen:

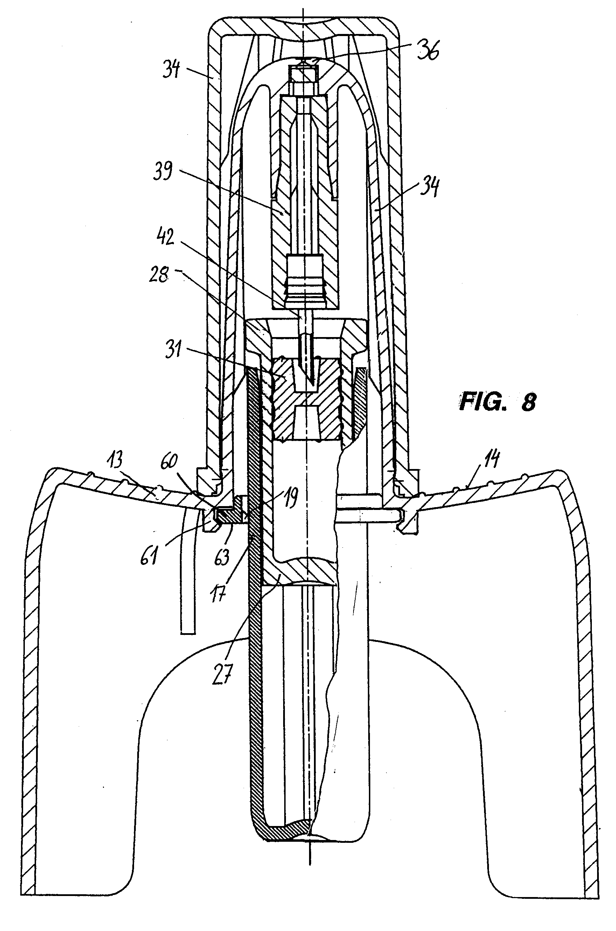

Die Materialbrücken 19 sind, wie Fig. 7 zeigt, als streifenförmige Stege ausgebildet und haben keine so ausgeprägte, nahezu punktförmige Sollbruchstelle, wie die dreiecksförmigen Materialbrücken entsprechend Fig. 2 und 3. Um trotzdem eine saubere und genaue Trennung zu erreichen, ist eine Schneidkante 55 vorgesehen, die im dargestellten Beispiel an einer unteren Innenkante des Stutzenabschnittes 34 angeformt ist. Dieser greift wie eine ringförmige Tasche über den Stutzen 18 des Basisgehäuseteiles 13 herüber.

Im unbetätigten Zustand liegt die Schneidkante 55 auf der Materialbrücke auf oder steht etwas darüber. Bei der Betätigung wird die Materialbrücke gegen die Schneidkante 55 gedrückt und abgeschnitten, was insbesondere dann vorteilhaft ist, wenn das Material eine große Bruchdehnung aufweist. Diese Schneidenausbildung könnte auch an anderen Bauteilen der Pumpe vorgesehen und auch bei anderen Formen der Materialbrücke eingesetzt werden. Wenn vorstehend jeweils drei Materialbrükken am Umfang gezeigt sind, so ist dies eine vorteilhafte Ausführung, die eine verkantungsfreie Betätigung ermöglicht, ohne daß zu viele Materialbrücken vorhanden sein müssen. Es kann aber auch eine andere Zahl gewählt werden.

Fig. 8 zeigt im Längsschnitt ein Detail einer Ausführungsform, bei der das Gehäuse 13 und der Stutzenabschnitt 34 einstückig ausgebildet sind. Im übrigen ist die Ausbildung ähnlich den Fig. 1 und 5. Es werden die gleichen Bezugszeichen für gleiche Teile verwendet und auf die vorhergehende Beschreibung dieser Teile und ihrer Funktion wird Bezug genommen.

An der Betätigungshülse 17, die den Behälter 27 aufnimmt, ist an der Außenseite ein Befestigungselement 60 in Form eines umlaufenden Ringes mit L-förmigem Querschnitt vorgesehen. Er ist mit der Betätigungshülse 17 über mehrere Materialbrücken 19 verbunden, die so ausgebildet und bemessen sind, daß sie unter dem Betätigungsdruck abreißen und somit den Ring von der Betätigungshülse trennen. Befestigungselement 60, Betätigungshülse 17 und Materialbrücken 19 sind einstückig aus Kunststoff gespritzt.

Das Befestigungselement 60 ist am Gehäuse 13 befestigt, und zwar durch eine Schnappverbindung 61. Das Befestigungselement zentriert sich mit seinem axial verlaufenden Schenkel an der Innenseite des Stutzenabschnitts 34, während der nach außen gerichtete Schenkel rastend in eine Vertiefung am Übergang zwischen der Betätigungsschulter 14 und dem Stutzenabschnitt 34 einrastet.

Bei der Montage wird die Betätigungshülse zusammen mit dem den Pumpenzylinder bildenden Behälter 27 eingebracht. Dabei kann ein Montagewerkzeug die in Fig. 11 untere Angriffsfläche 63 des Betätigungselementes 60 drücken, so daß die Einrastung der Rastverbindung 61 erfolgt, ohne daß man Gefahr läuft, daß die durch die Materialbrücken 19 gebildete Originalitätssicherung beschädigt wird. Wenn dagegen der Benutzer die Hülse nach oben bewegt, reißen die Materialbrücken 19 auf, und die Betätigungshülse kann zusammen mit dem Pumpenzylinder/Behälter 27 den Pumphub in der vorher beschriebenen Weise ausführen. Dabei führt sich die Betätigungshülse 17 einerseits in dem ringförmigen Befestigungselement 60 (zwischen den abgerissenen Materialbrücken) und andererseits an inneren Stegen 62 des Stutzenabschnitts 34. Auch ein Außenflansch des Behälter/Pumpenzylinders 27 wird so geführt. Dadurch wird ein Verkanten der Betätigung ausgeschlossen. Die Materialbrücken 19 können auch als eine geteilte oder durchgehende Filmverbindung ausgebildet sein.

Das Befestigungselement 60 hat in der Rastverbindung 61 axial und radial etwas Spiel. Dadurch ist es möglich, die Betätigungshülse 17 (beabsichtigt oder unbeabsichtigt) zu drehen, ohne die Originalitätssicherung zu zerstören. Dagegen sollte die Rastverbindung so kräftig sein, daß beim Abziehen der Betätigungshülse 17 nach unten die Originalitätssicherung reißt. Dies sichert den Inhalt des Dosierers vor unerwünschten Manipulationen am Inhalt.

Es ist hier zu erkennen, daß eine Originalitätssicherung geschaffen wird, die eine höhere, materialzerstörende Betätigungskraft erfordert, um den Medienspeicher 27 zu entsiegeln, als dies bei einer einfachen Nadel nötig wäre. Unterstützt wird dies durch die Reibungskraft zwischen Führungsflansch 47 und Haltelaschen 44, die, nachdem die Ruhereibung überwunden ist, in den Gleitzustand übergehen und daher einen größeren Anteil der aufgebrachten Betätigungskraft zur Betätigung und zur Überwindung der anderen Widerstände freigeben.

## Patentansprüche

1. Austragvorrichtung (11) für fließfähige Medien (29), mit einem Grundkörper (12) für die Aufnahme eines Medienspeichers und mit einer insbesondere für den Austrag in nur einem Hub ausgebildeten Schubkolbenpumpe, die einen an einer Kolbenlaufbahn (30) verschiebbar geführten Pumpkolben (31), der aus einem von einem Stößel (42) durchstoßbaren, einen Medienspeicher dicht verschließenden Kolbenstopfen besteht, sowie eine durch einen Pumpenzylinder (27) und den Pumpkolben (31) begrenzte, mit einer Austragöffnung (36) über einen Austragkanal (40) verbundene Pumpenkammer aufweist, wobei der Kolbenstopfen zusammen mit dem den Medienspeicher bildenden Pumpenzylinder (27) durch Ausübung einer Betätigungskraft auf eine Betätigungsfläche (25) gegen den Stößel bewegbar ist, **gekennzeichnet durch** wenigstens eine durch eine Betätigungskraft zerstörbare, eine Originalitätssicherung bildende und die ausreichende Betätigungskraft sicherstellende Materialbrücke (19, 32), wobei die Zerstörung der Materialbrücke einer Bewegung des Pumpkolbens (31) in dem Pumpenzylinder (27) vorauseilt.

2. Austragvorrichtung nach Anspruch 1, dadurch gekennzeichnet, daß die Materialbrücke (19) zwischen wenigstens einem mit dem Pumpenzylinder (27) bewegbaren Pumpenabschnitt (17, 27) und einem mit dem Grundkörper (12) verbundenen Gehäuseabschnitt (13) gebildet ist.

3. Austragvorrichtung nach Anspruch 2, dadurch gekennzeichnet, daß die Materialbrücke (19) durch einstückige Ausbildung von Gehäuse- und Pumpenabschnitt (13, 17) gebildet ist.

4. Austragvorrichtung nach Anspruch 2 oder 3, dadurch gekennzeichnet, daß an dem Gehäuse- oder Pumpenabschnitt (13, 17) an die Materialbrücke (19a) ein vorzugsweise ringförmiges Befestigungselement (60) angeformt ist, das an dem Pumpen- oder Gehäuseabschnitt (17, 13), insbesondere durch Einschnappen, angebracht ist.

5. Austragvorrichtung nach Anspruch 4, gekennzeichnet durch eine von dem Befestigungselement (60) und ggf. inneren Stegen eines Stutzenabschnitts (34) gebildete Führung für den Pumpenabschnitt (17).

6. Austragvorrichtung nach Anspruch 4 oder 5, dadurch gekennzeichnet, daß das Befestigungselement (60) eine Angriffsfläche (63) für ein Montageelement aufweist, das den Pumpenabschnitt in Rastmittel (61) am Gehäuseabschnitt (13) drückt.

7. Austragvorrichtung nach einem der Ansprüche 2 oder 3, dadurch gekennzeichnet, daß die Materialbrücke (19) im Inneren eines mit Betätigungsschultern (14) versehenen Basisgehäuseteils (13) ausgebildet ist.

8. Austragvorrichtung nach Anspruch 7, dadurch gekennzeichnet, daß das Basisgehäuseteil (13) mit einem die Pumpe zumindest teilweise aufnehmenden und die Austragöffnung (36) aufweisenden Stutzenabschnitt (34), vorzugsweise durch Verrasten, verbunden ist.

9. Austragvorrichtung nach einem der Ansprüche 2 bis 8, dadurch gekennzeichnet, daß die Materialbrücke (19) an eine den Pumpenzylinder (27) haltende und ggf. teilweise aufnehmende Betätigungshülse (17) angeformt ist.

10. Austragvorrichtung nach einem der Ansprüche 2 bis 9, dadurch gekennzeichnet, daß mehrere Materialbrücken (19) mit vorzugsweise gleichem Umfangsabstand voneinander im wesentlichen in einer Ebene vorgesehen sind.

11. Austragvorrichtung nach einem der Ansprüche 2 bis 10, dadurch gekennzeichnet, daß die wenigstens eine Materialbrücke (19) in Form einer mit einer Wandung verbundenen Spitze ausgebildet ist.

12. Austragvorrichtung nach einem der Ansprüche 2 bis 11, dadurch gekennzeichnet, daß die Materialbrücke (19) im Bereich einer oberen Kante einer Betätigungshülse (17) und einer nach unten weisenden Kante im Inneren des Basisgehäuseteiles (13) ausgebildet ist.

13. Austragvorrichtung nach einem der vorhergehenden Ansprüche, dadurch gekennzeichnet, daß an einem der bei der Betätigung der Pumpe zusammenwirkenden Teile, vorzugsweise am Grundkörper (12) eine Schneidkante (55) zum Durchtrennen einer Materialbrücke (19) vorgesehen ist, vorzugsweise an einer inneren unteren Kante eines Stutzenabschnittes (34).

14. Austragvorrichtung nach einem der vorhergehenden Ansprüche, dadurch gekennzeichnet, daß die Materialbrücke (32) durch einen Verschlußsteg im Bereich des den Pumpenkolben (31) bildenden Kolbenstopfens gebildet ist und der ihn durchstoßende Stößel (42) eine einen erhöhten Widerstand gegen das Durchstoßen erzeugende Stößelgestaltung hat.

15. Austragvorrichtung nach Anspruch 14, dadurch gekennzeichnet, daß der Stößel (42) seitliche Kanäle für die Leitung des Mediums aufweist und vorzugsweise einen kreuzförmigen Querschnitt aufweist.

16. Austragvorrichtung nach Anspruch 14 oder 15, dadurch gekennzeichnet, daß der Stößel (42) aus Kunststoff besteht, vorzugsweise am Grundkörper (12) bzw. einem damit verbundenen Kunststoffteil (48) angeformt ist und vor dem Durchstoßen eines Verschlußsteges im Kolben (31) mit seiner Außenfläche unter Abdichten der Pressung in eine Vertiefung (33) im Kolbenstopfen eindringt, wobei ggf. eine an den Stößel (42) anschließende Schulter (50) abdichtend auf einer Fläche (51) des Kolbens (31) aufliegt.

17. Austragvorrichtung nach einem der Ansprüche 14 bis 16, dadurch gekennzeichnet, daß der Stößel (42) an seiner Spitze eine doppelseitige Keilform aufweist und/oder einen Durchmesser hat, der größer als 1/6, vorzugsweise größer als 1/4, des Pumpenzylinderdurchmessers ist.

18. Austragvorrichtung nach einem der vorhergehenden Ansprüche, dadurch gekennzeichnet, daß an einem mit dem Pumpenzylinder (27) beweglichen Abschnitt, vorzugsweise an einer Betätigungshülse (17), eine mit einer Markierung an dem Grundkörper (12) zusammenwirkende Markierung vorgesehen ist, die bei Betätigung der Pumpe gegeneinander verschoben werden, wobei insbesondere die Markierungen zumindest zum Teil durch bauliche Gegebenheiten, wie Gehäusekanten oder dgl., gebildet sind.

19. Austragvorrichtung nach einem der vorhergehenden Ansprüche, dadurch gekennzeichnet, daß zwischen der Originalitätssicherungsstellung, in der die gegeneinander zur Betätigung bewegbaren Teile durch die Originalitätssicherung festgelegt sind, und der durch den Beginn des Medienaustrags bestimmten Austragbeginn-Stellung ein einen Leerweg definierender Abstand vorgesehen ist.

## Claims

1. Discharge apparatus (11) for flowable media (29), with a basic body (12) for the reception of a medium reservoir and with a thrust piston pump, particularly for the discharge in only a single stroke, which has a pump piston (31) displaceably guided on a piston runway (30), which comprises a piston plug tightly sealing a medium reservoir and perforatable by a ram (42), as well as a pump chamber bounded by a pump cylinder (27) and the pump piston (31) and connected to a discharge opening (36) by means of a discharge channel (40), the piston plug together with the pump cylinder (27) forming the medium reservoir being movable against the ram by exerting an actuating force on an actuating surface (25), characterized by at least one material bridge (19, 32) ensuring an adequate actuating force, which can be destroyed by an actuating force and forming a tamper-evident closure, the destruction of the material bridge preceding a movement of the pump piston (31) in the pump cylinder (27).

2. Discharge apparatus according to claim 1, characterized in that the material bridge (19) is formed between a pump portion (17, 27) movable with the pump cylinder (27) and a casing portion (13) connected to the basic body (12).

3. Discharge apparatus according to claim 2, characterized in that the material bridge (19) is formed by a one-piece construction of the casing and pump portions (13, 17).

4. Discharge apparatus according to claim 2 or 3, characterized in that onto a material bridge (19a) on the casing or pump portion (13, 17) is shaped a preferably circular fastening element (60), which is fitted to the pump or casing portion (17, 13), particularly by snapping in.

5. Discharge apparatus according to claim 4, characterized by a guide for the pump portion (17) formed by the fastening element (60) and optionally inner webs of a connector portion (34).

6. Discharge apparatus according to claim 4 or 5, characterized in that the fastening element (6) has an engagement face (63) for an assembly element, which presses the pump portion in latching means (61) on the casing portion (13).

7. Discharge apparatus according to one of the claims 2 or 3, characterized in that the material bridge (19) is constructed in the interior of a base casing part (13) provided with actuating shoulders (14).

8. Discharge apparatus according to claim 7, characterized in that the base casing part (13) is connected, preferably by locking, to a connector portion (34) at least partly receiving the pump and having the discharge opening (36).

9. Discharge apparatus according to one of the claims 2 to 8, characterized in that the material bridge (19) is shaped onto an actuating sleeve (17) retaining the pump cylinder (27) and optionally partly receiving the same.

10. Discharge apparatus according to one of the claims 2 to 9, characterized in that there are several material bridges (19) with preferably the same mutual circumferential spacing substantially in one plane.

11. Discharge apparatus according to one of the claims 2 to 10, characterized in that the at least one material bridge (19) is constructed in the shape of a tip connected to a wall.

12. Discharge apparatus according to one of the claims 2 to 11, characterized in that the material bridge (19) is constructed in the vicinity of an upper edge of an actuating sleeve (17) and a downwardly directed edge in the interior of the base casing part (13).

13. Discharge apparatus according to one of the preceding claims, characterized in that on one of the parts cooperating during pump actuation, preferably on the basic body (12), is provided a cutting edge (55) for cutting through a material bridge (19), preferably on an inner, lower edge of a connector portion (34).

14. Discharge apparatus according to one of the preceding claims, characterized in that the tamper-evident closure in the vicinity of a ram (42) piercing the piston plug forming the pump piston (31) is formed by a ram construction producing an increased resistance to piercing.

15. Discharge apparatus according to claim 14, characterized in that the ram (42) has lateral channels for conducting the medium and preferably has a cruciform cross-section.

16. Discharge apparatus according to claim 14 or 15, characterized in that the ram (42) is made from plastic and is preferably shaped onto the basic body (12) or a plastic part (48) connected thereto and prior to the piercing of a closure web in the piston (31) penetrates with its outer face and accompanied by pressure sealing a depression (33) in the piston plug and optionally a shoulder (50) connected to the ram (42) rests in sealing manner on one face (51) of the piston (31).

17. Discharge apparatus according to one of the claims 14 to 16, characterized in that the ram (42) has at its tip a double-sided wedge shape and/or a diameter which is larger than 1/6, preferably larger than 1/4 of the pump cylinder diameter.

18. Discharge apparatus according to one of the preceding claims, characterized in that on a portion movable with the pump cylinder (27), preferably on an actuating sleeve (17), is provided a marking cooperating with a marking on the basic body (12) and which are mutually displaced during pump actuation and in particular the markings are at least partly formed by constructional elements, such as casing edges or the like.

19. Discharge apparatus according to one of the preceding claims, characterized in that between the tamper-evident closure position in which the parts movable against one another for actuation are fixed by said closure and the discharge start position determined by the start of medium discharge, there is a spacing defining an idle path.

## Revendications

1. Dispositif verseur (11) pour produits coulants (29), comprenant un corps de base (12) pour le logement d'un réservoir de produit et une pompe à piston réalisant le déversement en une course et présentant un piston (31) guidé de manière coulissante sur une trajectoire de piston (30) et composé d'un bouchon de piston fermant de manière étanche un réservoir de produit et pouvant être transpercé par un poinçon (42), ainsi qu'une chambre de pompe reliée par un canal d'éjection (40) à une ouverture d'éjection (36) et limitée par un cylindre de pompe (27) et le piston de pompe (31), le bouchon de piston étant mobile à l'encontre du poinçon avec le cylindre de pompe (27) formant le réservoir de produit par application d'une force d'actionnement sur une surface d'actionnement (25), caractérisé au moins par un pont de matière (19, 32) destructible par une force d'actionnement, assurant la force d'actionnement suffisante et formant la garantie d'origine, la destruction du pont de matière précédant un mouvement du piston de pompe (31) dans le cylindre de pompe (27).

2. Dispositif verseur selon la revendication 1, caractérisé en ce que le pont de matière (19) est constitué entre au moins une section de pompe mobile (17, 27) avec le cylindre de pompe (27) et une section de boîtier (13) reliée au corps de base (12).

3. Dispositif verseur selon la revendication 2, caractérisé en ce que le pont de matière (19) est constitué par réalisation d'une seule pièce de la section de boîtier et de pompe (13, 17).

4. Dispositif verseur selon la revendication 2 ou 3, caractérisé en ce que sur la section de boîtier ou de pompe (13, 17) il est moulé sur le pont de matière (19a) un élément de fixation de préférence annulaire (60) qui est mis en place sur la section de pompe ou de boîtier (17, 13), en particulier par encliquetage.

5. Dispositif verseur selon la revendication 4, caractérisé par un guide de la section de pompe (17) formé par l'élément de fixation (60) et éventuellement par des listels internes d'une section de tubulure (34).

6. Dispositif selon la revendication 4 ou 5, caractérisé en ce que l'élément de fixation (60) présente une surface d'attaque (63) pour un élément de montage qui presse la section de pompe dans des crans d'arrêt (61) sur la section de boîtier (13).

7. Dispositif verseur selon l'une des revendications 2 ou 3, caractérisé en ce que le pont de matière (19) est ménagé à l'intérieur d'une partie de boîtier de base (13) munie d'épaulement d'actionnement (14).

8. Dispositif verseur selon la revendication 7, caractérisé en ce que la partie de boîtier de base (13) est reliée de préférence par encliquetage à une section de tubulure (34) logeant au moins partiellement la pompe et présentant l'ouverture d'évacuation (36).

9. Dispositif verseur selon l'une des revendications 2 à 8, caractérisé en ce que le pont de matière (19) est moulé à un manchon d'actionnement (17) maintenant et éventuellement logeant partiellement le cylindre de pompe (27).

10. Dispositif verseur selon l'une des revendications 2 à 9, caractérisé en ce que plusieurs ponts de matière (19) sont prévus sensiblement dans un plan, séparé les uns des autres à une distance périphérique de préférence identique.

11. Dispositif verseur selon l'une des revendications 2 à 10, caractérisé en ce qu'au moins un pont de matière (19) a la forme d'une pointe reliée à une paroi.

12. Dispositif verseur selon l'une des revendications 2 à 11, caractérisé en ce que le pont de matière (19) est formé dans la zone d'une bord supérieur d'un manchon d'actionnement (17) et d'un bord tourné vers le bas à l'intérieur d'une partie de boîtier de base (13).

13. Dispositif verseur selon l'une des revendications précédentes, caractérisé en ce que sur l'une des parties coopérant à l'actionnement de la pompe, de préférence sur le corps de base (12), il est prévu un bord de coupe (55) pour le sectionnement d'un pont de matière (19) de préférence sur un bord interne inférieur d'une section de tubulure (34).

14. Dispositif verseur selon l'une des revendications précédentes, caractérisé en ce que le pont de matière (32) est formé par un listel de fermeture dans la zone du bouchon de piston formant le piston de pompe (31) et le poinçon (42) perçant le bouchon a une configuration générant une résistance élevée à l'encontre du perçage.

15. Dispositif verseur selon la revendication 14, caractérisé en ce que le poinçon (42) présente des canaux latéraux pour l'acheminement du produit et de préférence une section transversale cruciforme.

16. Dispositif selon la revendication 14 ou 15, caractérisé en ce que le poinçon (42) se compose de matière synthétique, est moulé de préférence sur le corps de base (12) respectivement sur une partie en matière synthétique reliée (48) et avant la percée d'un listel de fermeture pénètre dans le piston (31) par sa surface externe rendue étanche sous la pression dans une cavité (33) dans le bouchon de piston, un épaulement (50) dans la continuité du poinçon (42) s'appliquant sur une surface (51) du piston (31) de manière étanche.

17. Dispositif verseur selon l'une des revendications 14 à 16, caractérisé en ce que le poinçon (42) présente à sa pointe une forme cunéiforme et/ou a un diamètre supérieur de 1/6, de préférence de 1/4 au diamètre du cylindre de pompe.

18. Dispositif verseur selon l'une des revendications précédentes, caractérisé en ce que sur une section mobile avec le cylindre de pompe (27), de préférence sur un manchon d'actionnement (17), il est prévu un marquage coopérant avec un marquage sur le corps de base (12) qui sont poussés l'un vers l'autre à l'actionnement de la pompe, les marquages étant constitués au moins en partie par des particularités de construction, tels que des bords de boîtier ou similaire.

19. Dispositif verseur selon l'une des revendications précédentes, caractérisé en ce qu'entre la position de garantie d'origine dans laquelle les parties mobiles l'une vers l'autre par l'actionnement sont déterminées par la garantie d'origine et la position de début d'évacuation définie par le début du déversement du produit, il est prévu une distance définissant une course à vide.
